# EUROPEAN PATENT SPECIFICATION

(11) **EP 2 491 877 B1**
(45) Date of publication and mention of the grant of the patent: **20.04.2016**
(21) Application number: 10824988.9
(22) Date of filing: 20.10.2010
(51) Int. Cl.: A61B 17/58, A61L 17/00

(54) **BRAIDED FLAT CABLE CONSTITUTED OF ULTRAHIGH-MOLECULAR POLYETHYLENE FIBERS**
AUS POLYETHYLENFASERN VON ULTRAHOHEM MOLEKULARGEWICHT ZUSAMMENGESETZTES GEFLOCHTENES FLACHKABEL
CÂBLE PLAT TRESSÉ CONSTITUÉ DE FIBRES DE POLYÉTHYLÈNE D'ULTRA-HAUTE MASSE MOLÉCULAIRE

(30) Priority: 22.10.2009 JP 2009243266
(43) Date of publication of application: 29.08.2012
(73) Proprietor: Alfresa Pharma Corporation, Chuo-ku Osaka-shi Osaka 540-8575 (JP)
(72) Inventor: UEDA, Yurito, Higashiosaka-shi Osaka 579-8036 (JP); MORIHARA, Tsutomu, Noda-shi Chiba 270-0216 (JP); SUDO, Toshio, Noda-shi Chiba 270-0216 (JP)
(74) Representative: Isarpatent
(86) International application number: PCT/JP2010/068514
(87) International publication number: WO 2011/049139

(56) References cited:
- EP-A2- 0 561 108
- WO-A1-2005/074827
- WO-A1-2008/018412
- WO-A1-2008/084669
- WO-A2-2007/071309
- US-A1- 2006 217 713

## Description

### TECHNICAL FIELD

The present invention relates to a method of making a surgical cable, in particular a flat cable for surgical use with which to firmly tie bones together and hold them in bone surgeries for treatment of bone fracture or repositioning and fixation of bones, and the like, so as to help the bones in the damaged region fuse and coalesce, or with which to suture soft tissues such as ligaments and the like, and more specifically a braided flat cable made of ultra-high molecular weight polyethylene fibers.

### BACKGROUND ART

In bone surgeries, such as for fixation (i.e., fusion and unification of bones) of repositioned bones after bone fracture, e.g., fracture of the spine, bone grafting, and the like, the bones, in order for their fixation, must be kept tightly held together, so that they may not be dislocated before their fusion is completed. For this holding, steel wires have long been used, along with, depending on situations, a variety of devices such as metal rods, hooks, bolts (pedicle screws) and the like. However, from a viewpoint of safety, such as avoiding of the risk of spinal cord being injured by fracture tips of steel wires which might be formed during or after a spine surgery, flat cables formed by braiding ultra-high molecular weight polyethylene fibers (molecular weight: not less than 400000), a type of polyethylene fibers, namely high strength fibers having high tensile strength and high tensile modulus of elasticity, have recently come to be widely used for tying bones, in place of steal wires, taking advantage of their high strength and flexibility [NESPLON™ Cable System, mftd by Alfresa Pharma Corp.] Making use of their high strength, the flat cables are also expected to come to be used for soft tissues, such as ligaments.

However, it has been found that ultra-high molecular weight polyethylene fiber is a material which could degrade, thought only slowly, through oxidation. That is, a test, in which flat cables formed by braiding fine fibers of ultra-high molecular weight polyethylene were implanted in rabbits for an extended period of time, revealed that such degradation and accompanying decline in strength are more likely to proceed if the flat cables contact with living tissues on the flat cables' surface which is widened due to expansion of their inter-fiber gaps (data not shown).

A flat cable for tying bones formed by braining ultra-high molecular weight polyethylene is an excellent material, with which it is possible to smoothly tie bones, tightly with a strong force. However, conventional flat cables formed by braiding ultra-high molecular weight polyethylene fibers shrink in their cross section area when a tensile force is applied to them, because the fibers are pulled to come into close together and their degree of congestion is increased, whereas in a relaxed state when they are released from the tensile force, their cross section expands because of expansion of the gaps between the fibers and decrease in the degree of their congestion. It was confirmed by the experiment described hereinafter that this is also the case with polyester fibers and is a common property among flat cables formed by braiding fibers, without regard to the type of the fibers. Because of increased contact of individual fibers with the living tissues, degradation of the fibers become faster when the cross section area of flat cables is expanded than when the cross section area of the flat cables is shrunk. Flat cables made of ultra-high molecular weight polyethylene fibers, when tied to bones in the body, are usually under a tensile force, but as the levels of the tensile force vary depending on the posture and motion of the body, there are also some occasions at which some part of the cable turns into a relaxed state. When relaxed and a cross section of the flat cables expands, with the gaps between the fibers expanding, the fibers come into wider contact with living tissues, thereby becoming more susceptible to degradation. This is still more important where the flat cables are applied to soft tissues, for which strong fastening is avoided. Therefore, it is desirable that surgical flat cables to be used in tying bones or suturing soft tissues like ligaments is those whose cross section area expands only a little even in a relaxed state. However, it was practically very difficult to produce such flat cables by braiding fibers.

Document WO2007071309 A2 discloses a method of making surgical cables according to the preamble of claim 1. DISCLOSURE OF INVENTION

### TECHNICAL PROBLEM

Against the above background, it is and objective of the present invention to provide a flat cable formed by braiding ultra-high molecular weight polyethylene fibers, characterized in that it is less likely to expand than conventional ones, with an elevated degree of congestions of fibers in a tension-free, relaxed state.

### SOLUTION TO PROBLEM

In a flat cable produced by braiding fibers, as the fibers cross with one another in directions diagonal to the longitudinal direction of the flat cable, forces are generated, when the flat cable is placed under a tensile force, in such a direction that the fibers come into close contact. Consequently, the gaps between the fibers reduce, and the cross section area of the flat cable shrinks under the tensile force. The present inventors studied for a method by which to produce a braided flat cable having reduced gaps between the fibers, using as an indicator the changes in the cross section area of the flat cable before and after loading a tensile force.

As a result, the present inventors found that by braiding ultra-high molecular weight polyethylene fibers into a flat cable and applying to it once a tensile stress of not less than a predetermined value for not shorter than a predetermined length of time, a braided flat cable is obtained which exhibits reduced expansion of the gaps between the fibers in a relaxed state, with the close contact of fibers of the flat cable substantially maintained even after release of the tensile stress. The present invention was completed through further studies based on this finding. Thus, the present invention provides what follows.
1. A flat cable made by braiding ultra-high molecular weight polyethylene fibers, wherein the cross section area of the cable under no load of tensile stress is not more than 110% of the cross section area under a load of a tensile stress of 22.7 N/mm².
2. The flat cable according to 1 above, which is for surgical use.
3. The flat cable according to 1 or 2 above, wherein the width thereof is 2-10 mm.
4. A method for production of a flat cable of braided ultra-high molecular weight polyethylene fibers, wherein the cross section area of the cable under no load of tensile stress is not more than 110% of the cross section area under a load of a tensile stress of 22.7 N/mm², comprising,
   a first process of braiding ultra-high molecular weight polyethylene fibers into a flat cable, and
   a second process of drawing the flat cable obtained in the said process up to a draw ratio of at least 109%.
5. The method for production according to 4 above, wherein the drawing is carried out at least for 30 seconds.
6. The method for production according to 4 or 5 above, which gives a flat cable for surgical use.

### EFFECTS OF INVENTION

The invention as defined above provides a method of making a braided flat cable made of ultra-high molecular weight polyethylene fibers, in which the gaps between the fibers are less likely to expand even in a state in which no tensile force is applied to the cable. The flat cable thus obtained is useful for preventing degradation, for even if some part of it is occasionally turns into a relaxed state depending on the posture or motion of the body after it is implanted in the body, with bones tied with it, the gaps between the fibers at the part are less likely to expand.

### BRIEF DESCRIPTION OF DRAWINGS

[Fig. 1] A photograph in substitution for a drawing, which shows product M at magnifications of 40X (left) and 100X (right).
[Fig. 2] A photograph in substitution for a drawing, which shows product N at magnifications of 40X (left) and 100X (right).
[Fig. 3] A photograph in substitution for a drawing, which shows product A at magnifications of 40X (left) and 100X (right).
[Fig. 4] A graph showing the change with time in the retention rate of tensile strength of the control cable and the cable of the present invention, after implanted in rabbits.
[Fig. 5] A graph showing the change with time in the retention rate of strength of the control cable and the cable of the present invention.

### THE BESTMODE FOR CARRING OUT THE INVNENTION

In the present invention, the term "flat" used of a cable means that its width is sufficiently greater than its thickness, and, for example, the width may be not less than 3 times the thickness, and generally is not less than 4 times.

The term "braid" used of cords or cables means a method in which many threads (each thread consisting of multiple ultra-high molecular weight polyethylene fibers in the present specification) are interweaved into a length of cable (braided cord), a method which is widely used for shoe straps, wire bundling tubing, and the like.

In the second process, in which the flat cable obtained in the first process by braiding ultra-high molecular weight polyethylene fibers is drawn, the term "draw ratio" means the ratio (%) of the length after the drawing to the length of the flat cable obtained in the first process before drawing.

Currently, NESPLON™ Cable System mentioned above is a commercially available flat cable for use in surgery, e.g., for tying bones, which is made by braiding ultra-high molecular weight polyethylene fibers, with two types of it available which differ in their width: one is about 3 mm and the other about 5 mm. As is described in the section of examples, a flat cable of about 3 mm in width (5000 d, d: denier*) formed by braiding ultra-high molecular weight polyethylene fibers can be drawn by suspending with it a weight having, e.g., 20 kg of mass, in which case a draw ratio of 109% was achieved in 30 seconds. The same tensile stress as the tensile stress occurring in the fibers as a whole in this case can also be applied for drawing of a flat cable with different width which is formed of the same fibers. For example, when a flat cable of about 5 mm in width (9900 d) is drawn, the mass of the weight to be employed is 20 × 9900/5000 = 39.6 kg. Therefore, for such a flat cable of 5 mm of width, suspension of a weight having at least this mass would cause to occur at least a comparable tensile stress, and drawing may be done with the weight so that a draw ratio of at least 109% is reached. Drawing is generally performed for not less than 30 seconds, which however is not requisite.
*) denier: number in gram per 9000 m of fiber(s)

Likewise, if a flat cable having a width other than 3 mm or 5 mm is prepared, the mass required for a weight may be calculated depending on its denier (d) value. The present invention thus can be properly applied also to flat cables having other width of from 2 to 10 mm, for example, which are formed by braiding ultra-high molecular weight polyethylene fibers. For drawing, not only such a method as using a weight, but also any mechanical means well known to skilled persons may be employed as desired.

In the present invention, the cross section area of a flat cable, in calculating the tensile stress of 22.7 N/mm², which is employed as the standard for determining how unlikely expansion occurs of the gaps between the fibers of the flat cable, is defined, for technical convenience, as the width × the thickness based on the measures of the flat cable in relaxed state before it is subjected to drawing.

### EXAMPLES

The present invention are described below in further detail with reference to examples. However, it is not intended that the present invention be restricted to those examples.

### (1) Study of the change in the cross section area of braided flat cables, as compared between when placed under no load of tensile force and placed under a load of a tensile force.

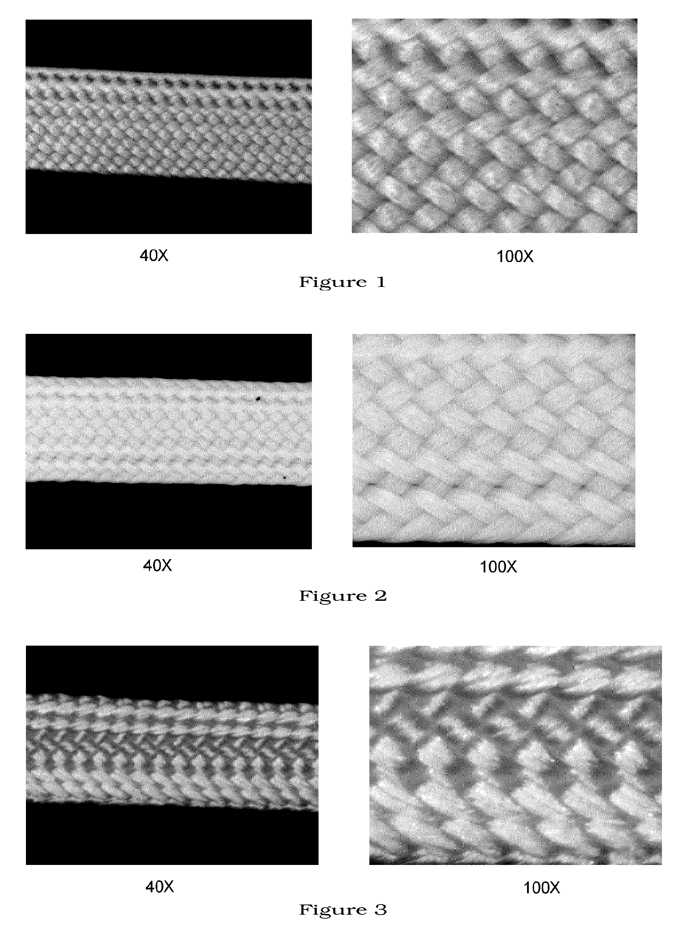

A preliminary test was carried out using flat cables formed by braiding synthetic fibers, without limiting fibers, to find out the amount of change in the cross section area when they are drawn. That is, using the following commercially available flat cables formed by braiding synthetic fibers, namely Wayolax™ tape with needle (braided cord of polyester with a needle, mftd by Matsuda Ika Kogyo, Co., Ltd., hereinafter referred to as "Product M": Fig. 1), Polyester Tape (tape of polyester, mftd by Nihon Chosen K. K., hereinafter referred to "Product N": Fig. 2), and NESPLON™ Cable System (ultra-high molecular weight polyethylene, mftd by Alfresa Pharma Corp.: hereinafter referred to "Product A", Fig. 3), their width and thickness were measured, both in a relaxed state under no load of tensile force and in a state under a load of a tensile force, to find out the ratio of the cross section area in the state under a load of a tensile force to the cross section area in the state under no load of tensile force. As the amount of the tensile force to be applied here, the value of 25.0 N was chosen, which is the average value applied per one cable when bones are tied with a flat cable for tying bones which is formed by braiding ultra-high molecular weight polyethylene fibers.

Specifically, for measurement under no load of tensile force, each sample was placed on a flat plate in such a manner that it is in contact with the plate with its wide surface, and the both ends of the region on which the measurement is to be made were fixed on the plate with cellophane tape. Using a CCD camera (Hi-Scope Advanced KH-3000, mftd by HIROX), the flat cable was photographed for its width from right above at a magnification of 40X, and for its thickness, from the side of it at a magnification of 100X, to determine the width and the thickness of the flat cable based on the images thus taken. The cross section area was calculated as the width times the thickness.

For measurement under a load of a tensile force, each sample was photographed likewise with the CCD camera to determine its width and thickness, with the sample being put around a metal round pipe with a smooth surface, under a tensile force of 25.0 N, and the cross section area was calculated likewise.

[Table 1]

**Table 1. Change in cross section area of commercially available flat cables made by braiding synthetic fibers**

| Sample/No. | | Under no load of tensile force | | | Under load of tensile force | | | Ratio of cross section area (%) no load/load |
|---|---|---|---|---|---|---|---|---|
| | | Width (mm) | Thickness (mm) | Cross section area (mm²) | Width (mm) | Thickness (mm) | Cross section area (mm²) | |
| Product M | 1 | 3.108 | 0.373 | 1.159 | 2.510 | 0.324 | 0.813 | 142.6 |
| | 2 | 3.167 | 0.387 | 1.226 | 2.559 | 0.333 | 0.852 | 143.8 |
| | Mean | 3.138 | 0.380 | 1.193 | 2.535 | 0.329 | 0.833 | 143.2 |
| Product N | 1 | 2.711 | 0.399 | 1.082 | 2.662 | 0.250 | 0.666 | 162.5 |
| | 3 | 2.985 | 0.382 | 1.140 | 2.657 | 0.265 | 0.704 | 161.9 |
| | 4 | 2.956 | 0.382 | 1.129 | 2.677 | 0.275 | 0.736 | 153.4 |
| | Mean | 2.884 | 0.388 | 1.117 | 2.665 | 0.263 | 0.702 | 159.3 |
| Product A | 1 | 3.098 | 0.559 | 1.732 | 2.583 | 0.505 | 1.304 | 132.8 |
| | 2 | 3.108 | 0.583 | 1.812 | 2.730 | 0.495 | 1.351 | 134.1 |
| | 3 | 3.044 | 0.554 | 1.686 | 2.760 | 0.490 | 1.352 | 124.7 |
| | Mean | 3.083 | 0.565 | 1.743 | 2.691 | 0.497 | 1.336 | 130.5 |

As seen in Table 1, it was found that each of the commercially available flat cables formed by braiding fibers changes in its cross section area by about 130-160% as compared between when placed under a load of a tensile force of 25.0 N and placed under no load of tensile force. The results indicate that the gaps between the fibers greatly expand in all the samples when placed under no load of tensile force.

### (2) Drawing of flat cables

By braiding 100-d ultra-high molecular weight polyethylene fibers into a tape form, a flat cable about 3 mm of width (5000 d) was produced, which then were divided by cutting them in proper length to prepare a number of pieces of flat cables with the same size and properties. Four of them were taken, and with each of them a weight having a mass of 20 kg was suspended (load of 196 N) for 15 seconds, 30 seconds, 1 minute, or 2 minutes, respectively, at room temperature, thus having prepared Samples 1-4. Each of these samples was measured for their length before and after the loading of the weight, and the draw ratio was calculated as "length after loaded/length before loaded". The results are shown in Table 2.

[Table 2]

**Table 2. Conditions for drawing of samples**

| | Sample 1 | Sample 2 | Sample 3 | Sample 4 |
|---|---|---|---|---|
| Duration of draw | 15 sec | 30 sec | 1 min | 2 min |
| Draw ratio | 1.088 | 1.090 | 1.093 | 1.098 |

### (3) Study of the change in the cross section area of flat cables with or without a load of a tensile force

The samples 1-4 obtained in (2) above and, as a control, an undrawn flat cable were measured for their cross section area when placed under no load of tensile force and under a load of a predetermined tensile force as follows.

Specifically, under no load of tensile force, each flat cable was placed on a flat plate in a manner that the former is in contact with the plate with its wide surface, and the both ends of the region on which measurement is to be made were fixed on the plate with cellophane tape. Using a CCD camera (Hi-Scope Advanced KH-3000, mftd by HIROX), each flat cable was photographed, at a magnification of 40X from right above for width, and at a magnification of 100X from the side for thickness, to determine the width and the thickness of the flat cable based on the images thus taken.

In measurement under a load of a tensile force, each sample or the control was put around a smooth round pipe (stainless) with a tensile force of 25.0 N, and photographed with the CCD camera for determination of the width and the thickness of the flat cable. Measurement was carried out 7 times on each sample and the control, and the cross section area was calculated for each measurement as the width times the thickness.

The largest and the smallest values were excluded from the values thus obtained of the cross section area, and the cross section area was determined as the average of the remaining 5 values, the results of which are shown in Table 3.

[Table 3]

**Table 3. Changes in cross section area of flat cables prepared by drawing under different conditions, placed under no load/load of tensile force ,**

| | | | Under no load of tensile force | | | Under load of tensile force | | | Ratio of cross section area (%) no load/load |
|---|---|---|---|---|---|---|---|---|---|
| | Duration of draw | Draw ratio (%) | Width (mm) | Thickness (mm) | Cross section area (mm²) | Width (mm) | Thickness (mm) | Cross section area (mm²) | |
| Control | 0 | - | 3.1257 | 0.5784 | 1.8080 | 2.7255 | 0.4739 | 1.2915 | 140% |
| Sample 1 | 15 sec | 1.088 | 2.4036 | 0.5036 | 1.2104 | 2.3382 | 0.4765 | 1.1141 | 109% |
| Sample 2 | 30 sec | 1.090 | 2.3434 | 0.4709 | 1.1036 | 2.2660 | 0.4566 | 1.0347 | 107% |
| Sample 3 | 1 min | 1.093 | 2.3307 | 0.4529 | 1.0556 | 2.2647 | 0.4475 | 1.0134 | 104% |
| Sample 4 | 2 min | 1.098 | 2.3392 | 0.4642 | 1.0859 | 2.3562 | 0.4621 | 1.0888 | 100% |

As shown in Table 3, with the control, the cross section area of the flat cable under no load of tensile force was 140% of that under a load of a tensile force, whereas with Sample 1, the change was greatly suppressed, to about 1/4, with the ratio of the cross section area being 109%. And the change rapidly became smaller as the draw ratio increases little by little: 107% with Sample 2, 104% with Sample 3, and 100% with Sample 4 (i.e., no change). From the results, it is understood that in a flat cable that has undergone drawing up to a draw ratio of not less than 1.088, the cross section area under no load of tensile force remains not more than 110% of the cross section area under a load of the tensile force, i.e., the increment in the cross section area remains not more than 10%. Moreover, it is expected that this value of not more than 10% increment in the cross section area can even more surely be achieved when the draw ratio is 1.090.

### (4) Test for confirmation of performance of a flat cable drawn in the same condition as the drawing of Sample 2

To confirm the above expectation, 10 flat cables (A1-A10) were provided which had undergone drawing in the same condition as in the drawing of Sample 2 (a weight of 20 kg, 30 sec), and each of them was measure for the cross section area both when placed under no load of tensile force and under a load of the tensile force of 25.0 N, in the same condition as in (3) above, to determine the ratio of the cross section area. The results are shown in Table 4.

[Table 4]

**Table 4. Changes in cross section are of flat cables prepared by drawing as Sample 2, placed under no load/load of tensile force**

| Sample | Under no load of tensile force | | | Under load of tensile force | | | Ratio of cross section area (%) no load/load |
|---|---|---|---|---|---|---|---|
| | Width (mm) | Thickness (mm) | Cross section area (mm²) | Width (mm) | Thickness (mm) | Cross section area (mm²) | |
| A1 | 2.4003 | 0.4628 | 1.1108 | 2.2598 | 0.4783 | 1.0809 | 103% |
| A2 | 2.3219 | 0.4557 | 1.0581 | 2.3023 | 0.4414 | 1.0162 | 104% |
| A3 | 2.3382 | 0.4718 | 1.1032 | 2.3186 | 0.4653 | 1.0789 | 102% |
| A4 | 2.3447 | 0.4854 | 1.1381 | 2.2990 | 0.4731 | 1.0877 | 105% |
| A5 | 2.3202 | 0.4887 | 1.1338 | 2.2533 | 0.4751 | 1.0705 | 106% |
| A6 | 2.3104 | 0.4401 | 1.0169 | 2.2516 | 0.4401 | 0.9910 | 103% |
| A7 | 2.3480 | 0.4803 | 1.1277 | 2.2549 | 0.4653 | 1.0493 | 107% |
| A8 | 2.3301 | 0.4912 | 1.1446 | 2.2304 | 0.4874 | 1.0870 | 105% |
| A9 | 2.3366 | 0.4492 | 1.0495 | 2.2859 | 0.4311 | 0.9854 | 107% |
| A10 | 2.3349 | 0.4524 | 1.0564 | 2.2843 | 0.4364 | 0.9969 | 106% |
| Mean | 2.3513 | 0.4689 | 1.1026 | 2.2949 | 0.4645 | 1.0659 | 105% |
| Standard deviation | 0.0236 | 0.0173 | 0.0428 | 0.0273 | 0.0192 | 0.0409 | 2% |

As seen in the results shown in Table 4, each of the flat cables which had been drawn in the same condition as Sample 2 exhibited a ratio of the cross section area between before and the loading the tensile force in the same condition as in (3) above, which was significantly lower than 110%, with the average of 105%. This indicates that in a flat cable the width of about 3 mm made by braiding ultra-high molecular weight polyethylene fibers, after having undergone drawing in the same condition as Sample 2, the cross section area when placed under no load of tensile force would steadily remain, at least, not more than 110% of that under the standard load of tensile force of 25.0 N. Since the cross section area (mean) of Samples A1-A10 under no load of tensile force is 1.1026 mm², while the tensile force loaded on them is 25.0 N, the tensile stress occurring in the cross section of the samples is 22.7 N/ mm² on an average. This indicates that the cross section area of a flat cable which has undergone drawing in the same condition as Sample 2 will surely remain, when placed in a relaxed condition (zero tensile force), not more than 110% of the cross section area measured under a tensile stress of 22.7 N/ mm² (provided that the cross section area here is the value under no load of tensile force), and that the expansion of the gaps between the fibers will be greatly suppressed.

### (5) Study of the changes in tensile strength of cables after implanted in animal bodies

In order to examine cables for change in their tensile strength after implanted in a relaxed state in the living body for a long period of time, the control cable (a conventional braided flat cable of ultra-high molecular weight polyethylene fibers which had undergone no drawing) and the Sample 2 cable, shown in Table 3, were implanted in muscles on the dorsal side of rabbits (6 locations per rabbit). A pair of samples (control and Sample 2) which had not been implanted but set aside were measured for the initial tensile strength. Number of the cables, control and Sample 2 cables, used in the test were both 13.

The samples were taken out sequentially 1, 3, 6 and 12 months after their implantation. Each sample, unimplanted or taken out, was measured for its tensile strength on a tensile testing machine equipped with chucks (RTC-1250A, A&D Co., Ltd.), with the distance between the chucks set at 50 mm and the pulling speed at 100 mm/mm. The results are shown below.

[Table 5]

**Table 5. Changes in strength of cables after implanted in animal bodies**

| Sample | Initial value | 1 month later | 3 months later | 6 months later | 12 months later |
|---|---|---|---|---|---|
| Control | 623 ± 15 N (100%) | 620 ± 69 N (100%) | 569 ± 38 N (91%) | 539 ± 42 N (87%) | 456 ± 34 N (73%) |
| Sample 2 | 700 ± 20 N (100%) | 703 ± 22 N (100%) | 690 ± 76 N (99%) | 638 ± 48 N (91%) | 544 ± 38 N (78%) |

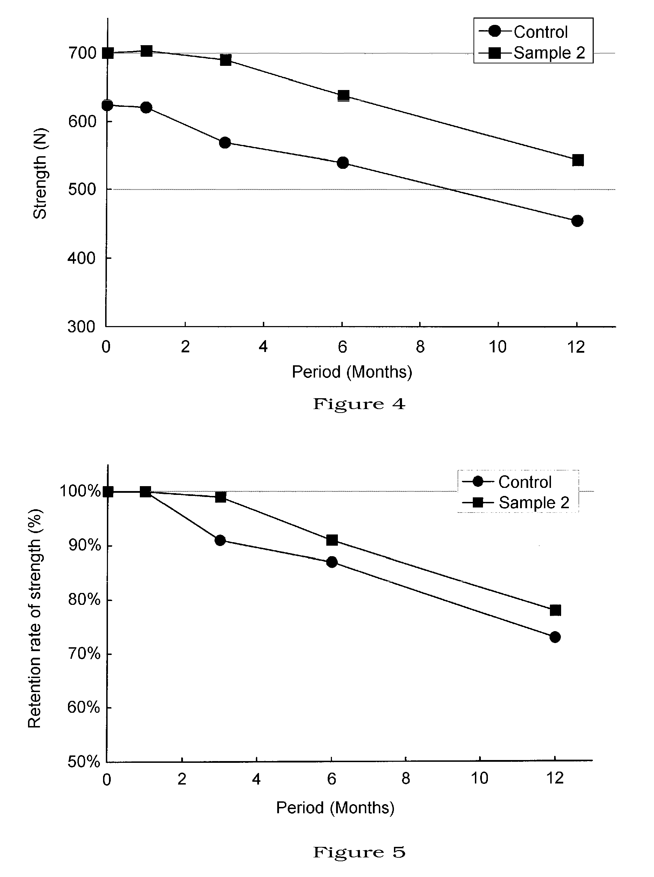

Table 5 shows the tensile strength of a samples before and after implantation for each indicated period of time, and the retention rate of tensile strength of the samples after implantation [expressed as (tensile strength of sample after implantation)/(initial tensile strength of sample) × 100], for both the control and Sample 2 cables. Fig. 4 is a graph representing the changes with time in tensile strength of both samples after implantation, and Fig. 5 the changes with time in the retention rate of tensile strength of both samples, respectively. Theses results show that the retention rate of tensile strength is higher in the Sample 2 cable. In particular, while a steep decline of tensile strength is observed in the control cable 3 months after implantation, no substantial decline is observed in the Sample 2 cable.

In many cases of bone surgeries for treatment of bone fracture and repositioning and fixation of bones, while it generally takes about 1 year for the bones in the damaged region to completely fuse and coalesce with one another, what is the most critical to achieve fusion and coalescence in the complete form as aimed is the quality in which the bones in the damaged region is held together during the period from just after the operation to about 3 months thereafter. Therefore, it is particularly desirable that the strength of the cable is maintained during this period. In this respect, the Sample 2 cable, whose initial strength was maintained 3 months after implantation, is excellent compared with the control cable, strength of which declined to 91% of the initial value.

Besides, as seen in the above results, the Sample 2 cable was found to have a higher initial tensile strength than the control cable. The Sample 2 cable is a cable prepared by subjecting the same cable as the control cable to a drawing process applying a tensile load of 196 N for 30 seconds. Since this was not a drawing performed in the process of production of the fibers, but a drawing which was carried out only after the cable was formed by braiding multiple strands consisting of multiple fibers, such a possibility is ruled out that the strength of each fiber was increased. Therefore, the reason is not known why the tensile strength of Sample 2 cable is greater than the control cable even in their initial values. It is assumed, however, that some changes which are favorable for mutual positioning or contact of fibers of the cable occurred during the drawing process, which have made the fibers less likely to break even under a strong tensile stress occurring during a tensile strength measurement.

### INDUSTRIAL APPLICABILITY

The braided flat cable of ultra-high molecular weight polyethylene fibers obtained according to the present invention is a cable in which the gaps between the fibers are less likely to expand even in a relaxed state, and, therefore, after it is implanted in the body with bones tied with it, even if some part of it is occasionally relaxed depending on the posture or motion of the body, its degradation in the body is suppressed, and thus increased safety is provided, because substantial surface of contact of such a part with the living tissues is reduced compared with conventional cables.

## Claims

1. A method for production of a flat cable for surgical use of braided ultra-high molecular weight polyethylene fibers, wherein the cross section area of the cable under no load of tensile stress is not more than 110% of the cross section area under a load of a tensile stress of 22.7 N/mm², comprising,
a first process of braiding ultra-high molecular weight polyethylene fibers into a flat cable, and
a second process of drawing the flat cable obtained in the said process up to a draw ratio of at least 109%,
wherein draw ratio means the ratio (%) of the length after the drawing to the length of the flat cable obtained in the first process before drawing,
**characterised in that** the second process takes place at room temperature.

2. The method for production according to claim 1, wherein the drawing is carried out at least for 30 seconds.

## Patentansprüche

1. Verfahren zur Herstellung eines flachen Kabels zur chirurgischen Verwendung aus geflochtenen Polyethylenfasern mit ultrahohem Molekulargewicht, wobei der Querschnittsbereich des Kabels ohne Zugspannungsbelastung nicht mehr als 110% des Querschnittsbereichs unter Zugspannungsbelastung von 22,7 N/mm² beträgt, umfassend:
einen ersten Prozess des Flechtens von Polyethylenfasern mit ultrahohem Molekulargewicht zu einem flachen Kabel, und
einen zweiten Prozess des Ziehens des flachen Kabels, das in dem Prozess erhalten wurde, bis zu einem Ziehverhältnis von wenigstens 109%, wobei das Ziehverhältnis das Verhältnis (%) der Länge nach dem Ziehen zur Länge des flachen Kabels, das in dem ersten Prozess erhalten wurde, vor dem Ziehen bedeutet,
**dadurch gekennzeichnet, dass** der zweite Prozess bei Raumtemperatur stattfindet.

2. Herstellungsverfahren nach Anspruch 1, wobei das Ziehen wenigstens 30 Sekunden lang durchgeführt wird.

## Revendications

1. Procédé de production d'un câble plat pour un usage chirurgical de fibres de polyéthylène de ultra-haut poids moléculaire tressées, dans lequel l'aire de section transversale du câble sans charge de résistance à la traction est de pas plus de 110 % de l'aire de section transversale sous une charge d'un effort de traction de 22,7 N/mm², comprenant :
un premier processus de tressage de fibres de polyéthylène de ultra-haut poids moléculaire dans un câble plat, et
un deuxième processus d'étirement du câble plat obtenu dans ledit processus jusqu'à un rapport d'étirement d'au moins 109 %,
où le rapport d'étirement signifie le rapport (%) de la longueur après l'étirement du câble plat obtenu dans le premier processus avant l'étirement,
**caractérisé en ce que** le deuxième processus a lieu à température ambiante.

2. Procédé de production selon la revendication 1, dans lequel l'étirement est réalisé durant au moins 30 secondes.
